# FASCICULE DE BREVET EUROPEEN

(11) **EP 4 472 727 B1**
(45) Date de publication et mention de la délivrance du brevet: **19.11.2025**
(21) Numéro de dépôt: 23702472.4
(22) Date de dépôt: 02.02.2023
(51) Int. Cl.: A61N 1/36, A61N 1/40, A61N 1/04

(54) **DISPOSITIF D'ÉLECTROTHÉRAPIE HAUTE FRÉQUENCE BI-CANAL SÉRIALISÉS OU PARALLÉLISÉS**
HOCHFREQUENZ-ELEKTROTHERAPIEGERÄT MIT ZWEI KANÄLEN IN SERIE ODER PARALLEL
HIGH-FREQUENCY ELECTROTHERAPY DEVICE HAVING TWO CHANNELS IN SERIES OR IN PARALLEL

(30) Priorité: 03.02.2022 FR 2200975; 03.02.2022 FR 2200974
(43) Date de publication de la demande: 11.12.2024
(73) Titulaire: Winback Group, 64200 Biarritz (FR)
(72) Inventeur: BUÉE, Christophe, 64500 Saint Jean de Luz (FR); JOVÈNE, Gilles, 06700 St Laurent du Var (FR); BROUCHET, Sebastien, 06310 Beaulieu sur mer (FR); JEAMMOT, Guillaume, 06200 Nice (FR)
(74) Mandataire: Hautier IP
(86) Numéro de dépôt international: PCT/EP2023/052601
(87) Numéro de publication internationale: WO 2023/148288

(56) Documents cités:
- US-A- 5 776 173
- US-A1- 2003 181 960
- US-A1- 2010 152 817
- US-A1- 2017 001 004

## Description

### DOMAINE TECHNIQUE

La présente description concerne un appareil électronique pour un usage thérapeutique ou cosmétique. L'enseignement correspondant trouvera plus particulièrement son application dans le domaine dit de l'électrothérapie notamment pour le traitement par diathermie et/ou conductivité.

### ETAT DE LA TECHNIQUE

L'électrothérapie est une technique non invasive, sans danger qui consiste à employer de l'électricité dans un but thérapeutique. Cette technique est reconnue pour soulager la douleur, renforcer les fibres musculaires ou bien encore accélérer la cicatrisation des tissus biologiques. 3 grandes familles de fréquences existent comme les basses fréquences (1 Hz - 150 Hz) pour une action neurostimulante superficielle, les moyennes fréquences (1 kHz - 1kHz) pour une action neurostimulante profonde et enfin les hautes fréquences (100 kHz - 1,2 MHz) pour une action diathermique sélective superficielle ou profonde et une accélération de la cicatrisation. Les basses fréquences (BF), moyennes fréquences (MF) sont désignées généralement comme électrostimulation et les hautes fréquences (HF) sont désignées comme radiofréquence.

Ces différents types de courants d'électrothérapie circulent entre deux plaques ou éléments conducteurs qui jouent le rôle d'électrodes au contact de la peau. Différents types de courants peuvent être utilisés par le praticien.

La chaleur est une modalité thérapeutique utilisée depuis de nombreuses années en kinésithérapie et divisée en deux catégories : les agents chauffants superficiels et les agents chauffants profonds. Les modalités de thermothérapie profonde incluent la diathermie thérapeutique ou cosmétique à ondes longues et à ondes courtes, les ultrasons, et la radiofréquence de contact, cette dernière étant appelée aussi courant de haute fréquence qui est entre 100 kHz et 1,2 MHz. Ce type de thermothérapie profonde est appelé diathermie. La diathermie crée un échauffement dans les tissus cellulaires des parties lésées du corps entre deux électrodes en contact avec le tissu vivant, de sorte qu'une circulation de courant électrique se produit dans le corps entre ces deux électrodes.

Par définition, l'équipement de diathermie par conduction de courant comporte une électrode active et une électrode de retour, comme divulgué par exemple, dans le brevet ES 287 964.

En raison de l'impédance électrique du tissu lui-même, le courant électrique circule à travers le tissu et provoque une élévation de la température du tissu par effet Joule. Cet échauffement est important et est lié à l'augmentation de l'intensité du courant.

La conductivité du tissu évolue en fonction du courant à haute fréquence. Plus sa tension est importante, plus le tissu sera conducteur et accélèrera sa cicatrisation.

Lorsqu'il y a plusieurs électrodes, elles sont connectées à un seul générateur de tensions haute fréquence ce qui favorise l'effet diathermie et la cicatrisation des tissus en même temps dans le traitement. Ces deux effets sont donc combinés et ne peuvent être dissociés.

On connait le document US 5776173 A1 qui propose un appareil de stimulation interférentielle pour électrothérapie. L'appareil comprend deux oscillateurs générant chacun un signal de sortie ayant une fréquence, le signal de sortie passe par plusieurs composants avant de passer par un mélangeur. Le mélangeur combine les signaux de sortie qui sont ensuite appliqués à un commutateur qui sélectionne une modalité de sortie selon une thérapie bipolaire ou quadripolaire. Cet appareil fournit un seul signal mixé et basse fréquence à la paire d'électrodes.

On connait le document US 2010/0152817 A1 qui propose un simulateur de réseau pour la stimulation électrique des nerfs par des paires d'électrodes successivement ou alternativement selon différents modes d'application. Le simulateur produit des impulsions électriques de telle sorte que les signaux sont soit transmis séquentiellement à des paires d'électrodes successives dans un cycle de sorte que les paires d'électrodes respectives reçoivent les signaux correspondants à des moments différents, soit alternativement de sorte que les signaux soient reçus par les paires d'électrodes de sorte qu'ils ne commencent et ne se terminent pas tous en même temps. Ce simulateur propose grâce à des applications variées d'impulsions électriques de générer une impulsion de simulation plus longue ou d'amplitude plus élevée. Ce simulateur fournit un même signal à chaque paire d'électrodes.

On connait également le document US 2003/0181960 A1 qui propose un appareil d'électrothérapie qui à partir d'un premier signal et d'un deuxième signal génère un signal thérapeutique administré par une électrode et récupéré par une électrode de retour. Cet appareil ne permet pas d'administrer des traitements variés. Cet appareil fonctionne dans les basses fréquences.

Il existe donc le besoin de proposer une solution qui permet des traitements optimisés notamment moins risqués et plus efficaces pour l'utilisateur qui reçoit le traitement et simplifiés pour le praticien qui administre le traitement.

Les autres objets, caractéristiques et avantages du présent enseignement apparaîtront à l'examen de la description suivante et des dessins d'accompagnement. Il est entendu que d'autres avantages peuvent être incorporés.

### RESUME

Pour atteindre cet objectif, selon un mode de réalisation on prévoit un dispositif d'électrothérapie comprenant :
- N électrodes, N étant égal à 2 ou 3,
- deux générateurs de tension sinusoïdale, avantageusement destiné à produire respectivement un premier signal et un deuxième signal,
   caractérisé en ce que
- le dispositif comprend un transformateur pour chaque générateur de tension de sorte que les deux générateurs de tension sont isolés par un transformateur et
- le dispositif comprend une unité de commande configurée pour que le dispositif prenne alternativement :
   - une première configuration dite en série dans laquelle les N égal à 2 électrodes étant des électrodes actives et les deux générateurs de tension sont mis en série, le dispositif ne comprend pas d'électrode de retour, chaque électrode active étant connectée à un générateur de tension, le dispositif est configuré pour générer un potentiel différent à chaque électrode active de sorte à assurer la transmission du courant entre deux électrodes actives les plus près,
   - une deuxième configuration dite en parallèle dans laquelle N égale à 3, dont deux électrodes sont des électrodes actives chacune respectivement connectée à un générateur de tension et une électrode est une électrode de retour pour former la masse et est connectée à chaque générateur de tension, les deux générateurs de tension sont mis en parallèle, le dispositif est configuré pour générer un potentiel différent à chaque électrode active de sorte à assurer la transmission du courant entre deux électrodes actives les plus près et/ou entre chaque électrode active et l'électrode de retour.

Avec ce dispositif, il est donc possible d'appliquer plusieurs signaux au travers de plusieurs électrodes sur le corps de l'utilisateur, c'est un dispositif bi-canal. Le praticien peut réaliser un traitement complet proposant la diathermie et/ou la conductivité en parallélisant ou en sérialisant les deux générateurs de tension. La présence de deux générateurs isolés assure une utilisation sans risque pour l'utilisateur qui est isolé sur circuit électrique. Par ailleurs, la possibilité de jouer sur les courants dérivés entre les électrodes actives pour assurer le traitement de l'utilisateur permet d'augmenter les surfaces de zones traitées et ainsi de proposer un traitement multiple. Ce choix est assez surprenant, car classiquement on cherche à réduire les courants dérivés entre électrodes actives pour focaliser sur les courants entre électrodes active et électrode de retour ou neutre.

Avantageusement, chaque électrode active est connectée à un générateur de tension de sorte à recevoir respectivement le premier signal ou le deuxième signal produit par chaque générateur de tension. Préférentiellement, le premier signal et le deuxième signal sont différents.

Un autre aspect concerne un procédé de fonctionnement d'un dispositif tel que décrit ci-dessus comprenant :
- un premier mode de fonctionnement selon la première configuration du dispositif dans lequel N électrodes, N étant égal à 2, sont actives chacune connectée à un générateur de tension, les deux générateurs de tension sont connectés en série, le courant circulant entre les deux électrodes actives,
- un deuxième mode de fonctionnement selon la deuxième configuration du dispositif dans lequel N étant égal à 3, dont 2 électrodes sont actives chacune connectée à un générateur de tension, une électrode est une électrode de retour, les deux générateurs de tension sont connectés en parallèle, le courant circulant entre les électrodes actives les plus proches et entre les électrodes actives et l'électrode de retour.

### BREVE DESCRIPTION DES FIGURES

Les buts, objets, ainsi que les caractéristiques et avantages du présent enseignement ressortiront mieux de la description détaillée d'un mode de réalisation de cette dernière qui est illustré par les dessins d'accompagnement suivants dans lesquels :
La figure 1 représente le schéma électrique d'un dispositif selon un premier mode de réalisation suivant la première configuration à deux électrodes actives et deux générateurs de tensions.
La figure 2 représente la circulation des courants entre les électrodes d'un dispositif selon la figure 1 appliquée sur un corps humain.
La figure 3 représente le schéma électrique d'un dispositif selon un premier mode de réalisation de l'invention suivant la deuxième configuration à deux électrodes actives, une électrode neutre de retour et deux générateurs de tension.
La figure 4 et la figure 5 représentent la circulation des courants entre les électrodes d'un dispositif selon la figure 3 appliquée sur un corps humain. La figure 4 illustre la circulation des courants entre deux électrodes actives lors de la synchronisation des générateurs. La figure 5 illustre la circulation des courants entre deux électrodes actives et entre les électrodes actives et l'électrode de retour lors de la désynchronisation des générateurs.
La figure 6 représente le schéma électrique d'un dispositif selon un deuxième mode de réalisation de l'invention suivant la première configuration, à deux électrodes actives étant également électrode de retour et deux générateurs de tensions.
La figure 7 représente la circulation des courants d'un dispositif selon la figure 6 appliquée sur le corps humain.

### DESCRIPTION DÉTAILLÉE

Avant d'entamer une revue détaillée de modes de réalisation, sont énoncées ci-après des caractéristiques optionnelles qui peuvent éventuellement être utilisées en association ou alternativement.

Selon un exemple, chaque générateur de tension est configuré pour générer une tension haute fréquence, préférentiellement entre 100 kHz et 10 MHz.

Selon un exemple, les électrodes actives sont configurées pour être mobiles.

Selon un exemple, les électrodes actives sont capacitives ou résistives ou multipolaires.

Selon un exemple, chaque générateur de tension comprend un organe de mesure configuré pour mesurer des paramètres de sortie du générateur de tension.

Selon un exemple, chaque générateur de tension comprend un module de contrôle configuré pour contrôler le courant de sortie et/ou la fréquence et/ou le déphasage du générateur de tension. Le module de contrôle est par exemple un microcontrôleur ou un microprocesseur ou un circuit logique programmable (CPLD Complex Programmable Logic Device) ou un réseau de portes programmables in situ (Field Programmable Gate Array FPGA) ou un circuit analogique. Selon un exemple, l'organe de mesure communique avec le module de contrôle pour lui fournir des données nécessaires au contrôle. Avantageusement, le générateur de tension peut contrôler sa sortie en fonction de l'impédance mesurée par l'organe de mesure.

Selon un exemple, le dispositif comprend un organe de synchronisation configuré pour contrôler la synchronisation ou la désynchronisation des générateurs de tension. Selon un exemple, le dispositif ne comprend pas de commutateur agencé entre une sortie d'un générateur de tension et une électrode active.

Selon un exemple, le dispositif ne comprend pas d'organe de mixage agencé entre une sortie d'un générateur de tension et une électrode active.

Selon un exemple, le procédé comprend une étape de contrôle du phasage ou déphasage des deux générateurs avantageusement par le module de contrôle, et comprenant une étape de contrôle de la synchronisation par l'émission d'un signal de synchronisation ou désynchronisation des deux générateurs avantageusement par l'organe de synchronisation de sorte à générer un courant circulant entre les deux électrodes actives.

La présente description concerne un dispositif d'électrothérapie apte à fournir un traitement par diathermie et/ou conductivité du corps de l'utilisateur.

Avantageusement, le dispositif d'électrothérapie selon le présent enseignement permet de traiter le corps de l'utilisateur par diathermie ou conductivité ou bien par diathermie combinée à la conductivité.

Avantageusement, les différents traitements sont réalisés par la mise en série, que l'on nomme également sérialisation, ou la mise en parallèle, que l'on nomme également parallélisation, des générateurs de tensions du dispositif.

Le dispositif d'électrothérapie comprend plusieurs électrodes, c'est-à-dire un nombre d'électrodes égal à deux ou trois. On définit préférentiellement le nombre d'électrodes du dispositif par le nombre N, N étant égal à 2 ou 3. Selon une possibilité, le nombre d'électrodes est égal à deux ou trois ou plus généralement à un nombre pair ou impair.

Le dispositif d'électrothérapie comprend également deux générateurs de tension sinusoïdale 210, 220.

Préférentiellement, on définit le nombre de générateurs de tension sinusoïdale 210, 220 du dispositif par rapport au nombre d'électrodes et plus particulièrement les électrodes actives 212, 222. Selon une possibilité dans laquelle le nombre d'électrodes actives est un nombre pair, le nombre de générateur de tension sinusoïdale est égal à ce nombre d'électrodes.

Le dispositif comprend deux canaux, chaque canal est issu d'un générateur de tension 210, 220. Selon une possibilité, chaque générateur de tension 210, 220 comprend au moins un canal dit canal d'émission correspondant à une sortie du signal du générateur. Les électrodes sont des électrodes actives 212, 222, c'est-à-dire que l'électrode fournit un courant au corps de l'utilisateur 10. Préférentiellement, les deux électrodes actives 212, 222 sont connectées un canal différent. Avantageusement, chaque électrode active 212, 222 est connectée à un générateur de tension, plus précisément à un canal d'émission d'un générateur de tension. Le dispositif peut également comprendre selon les configurations une électrode de retour 240 ou neutre, c'est-à-dire une électrode recevant le courant émis par les électrodes actives 212, 222 et ayant traversé une portion du corps de l'utilisateur 10. Selon une possibilité, chaque générateur de tension 210 , 220 comprend un canal dit canal de réception correspondant à une entrée recevant le courant ayant traversé une portion du corps de l'utilisateur 10. L'électrode de retour 240 assure la fermeture du circuit électrique au niveau du corps de l'utilisateur 10. L'électrode de retour 240 forme la masse. L'électrode de retour 240 est préférentiellement fixe, mais peut être mobile selon les traitements. On entend par fixe que pendant le traitement l'électrode de retour n'est pas déplacée par le praticien, l'électrode peut être maintenue fixe sur l'utilisateur par des moyens de maintien. On entend par mobile que pendant le traitement l'électrode est déplacée par le praticien.

Les électrodes 212, 222, 240 sont configurées pour être appliquées sur le corps de l'utilisateur 10. Avantageusement, les électrodes 212, 222, 240 sont en contact avec le corps de l'utilisateur 10.

Les électrodes actives 212, 222 peuvent être capacitives, résistives, multipolaires. On entend par multipolaire que l'électrode active 212, 222 comprend deux pôles conducteurs, tel qu'un pôle capacitif et un pôle résistif simultanément.

Les électrodes actives 212, 222 peuvent être fixes ou mobiles selon les besoins du traitement.

Le dispositif d'électrothérapie selon le présent enseignement comprend également une unité de commande configurée pour que le dispositif prenne alternativement une première configuration et une deuxième configuration. L'unité de commande est par exemple un microcontrôleur ou un microprocesseur ou un circuit logique programmable (CPLD Complex Programmable Logic Device) ou un réseau de portes programmables in situ (Field Programmable Gate Array FPGA) ou un circuit analogique.

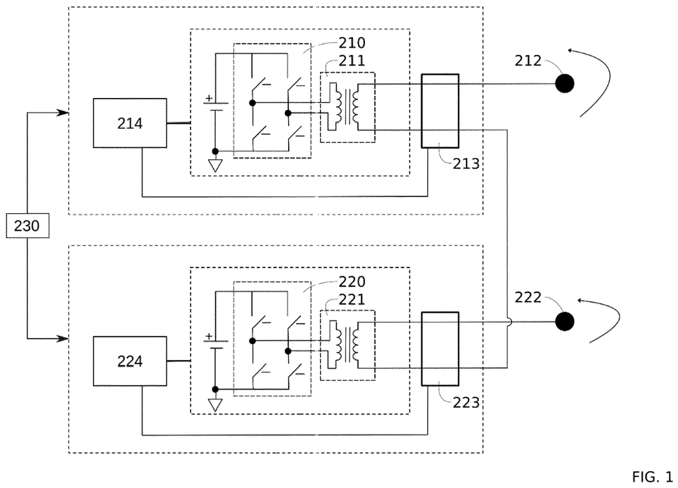

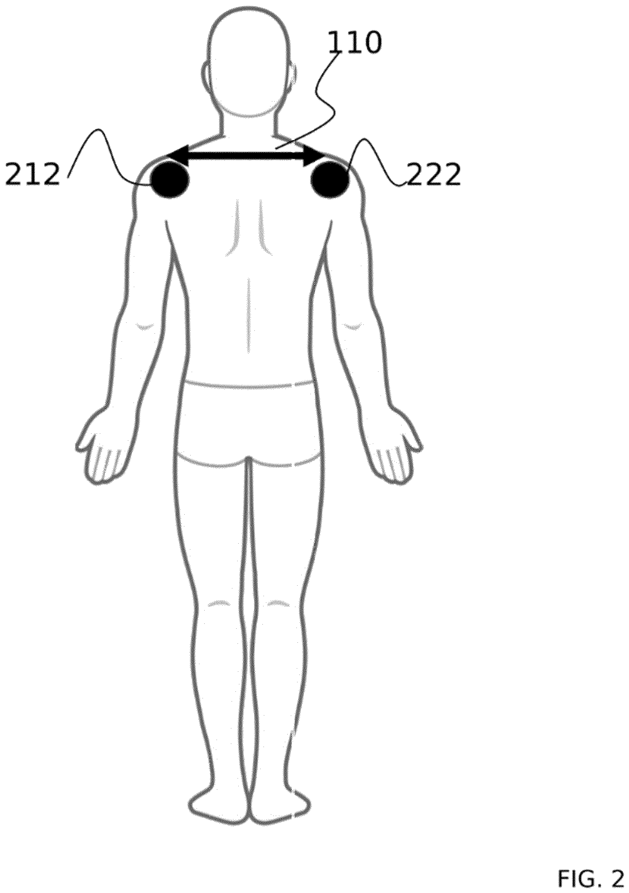

Selon une première configuration, le dispositif comprend N électrodes, avec N égal à 2, qui sont des électrodes actives 212, 222, le dispositif ne comprend pas d'électrode de retour 240. Ainsi, chaque électrode 212, 222 est connectée à un générateur de tension 210, 220. Les deux générateurs de tension 210, 220 sont agencés en série. Cette configuration est illustrée à la figure 1 ainsi qu'à la figure 2.

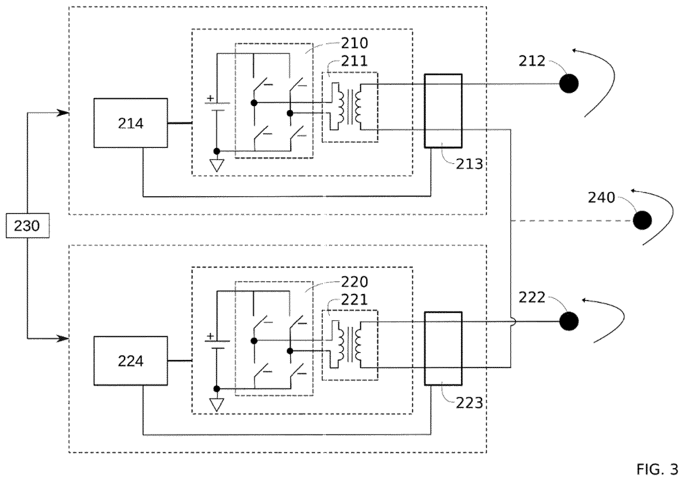

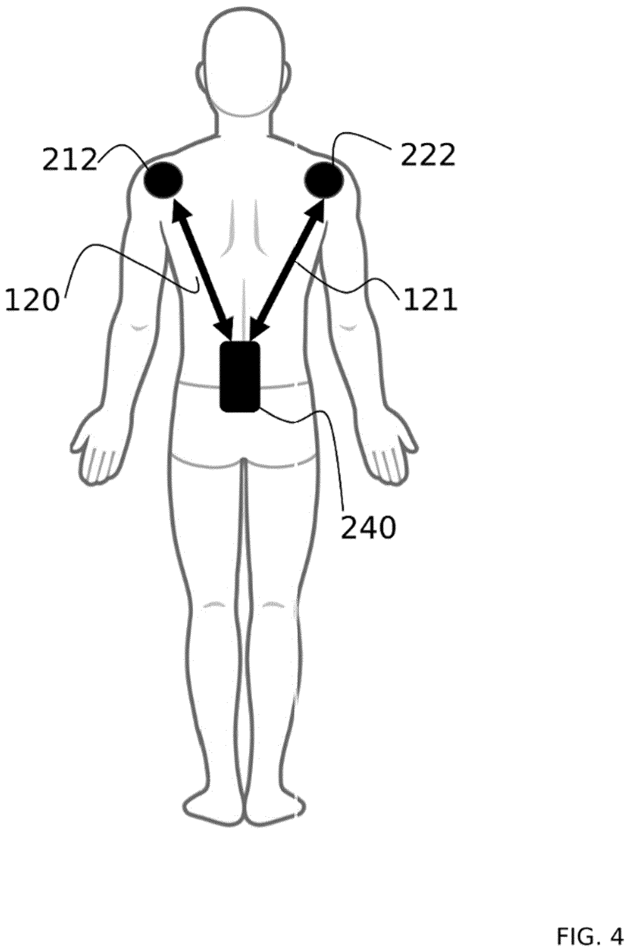

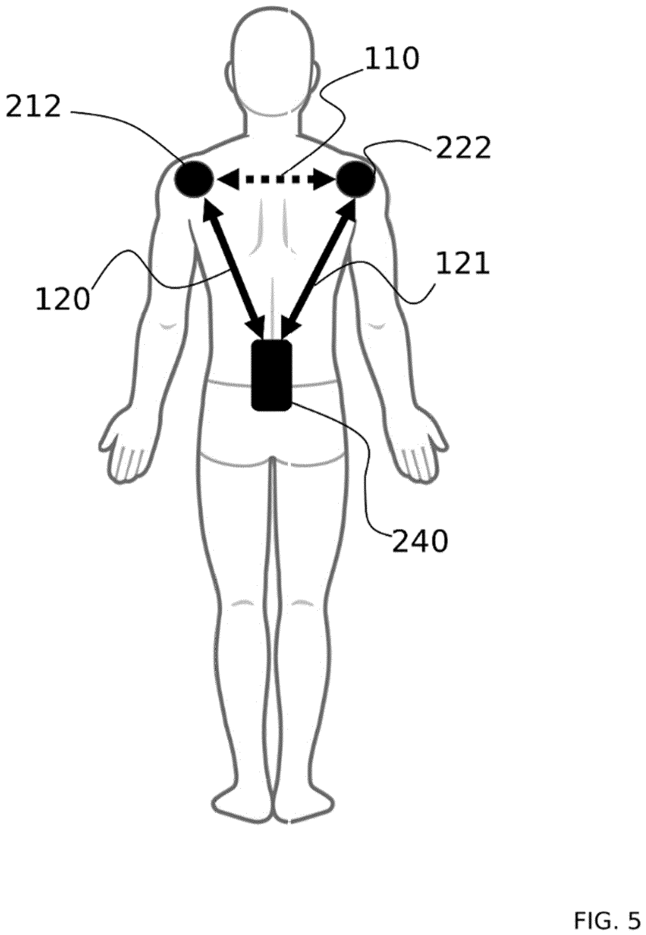

Selon une deuxième configuration, le dispositif comprend N électrodes, avec N égal à 3, dont deux électrodes sont des électrodes actives 212, 222 et une électrode est une électrode de retour 240. Ainsi chaque électrode active 212, 222 est connectée à un générateur de tensions 210, 220. L'électrode de retour 240 est connectée au point commun des deux générateurs de tensions 210, 220. Préférentiellement, le point commun des deux générateurs de tension 210, 220 est connecté à chacun des canaux de réception de chaque générateur. Les deux générateurs de tension 210, 220 sont agencés en parallèle. Cette configuration est illustrée à la figure 3 ainsi qu'aux figures 4 et 5.

Selon un mode de réalisation préféré, les générateurs de tension 210, 220 sont isolés. Les générateurs de tension 210, 220 sont isolés galvaniquement. Selon un mode de réalisation représenté sur les figures, l'isolation des générateurs de tension 210, 220 est matérialisée par un transformateur 211, 221 agencé en sortie de chaque générateur de tension 210, 220. Le générateur de tension isolé permet d'assurer une grande sécurité pour l'utilisateur qui reçoit un courant tout en étant isolé du circuit électrique du dispositif.

Les transformateurs 211, 222 sont avantageusement configurés pour être en phase ou en déphasage notamment en opposition selon le besoin que les signaux émis par les générateurs de tension 210, 220 soient en phase ou en déphasage.

Selon le présent enseignement, le dispositif permet ainsi d'utiliser ou non une électrode de retour 240. Le dispositif selon l'invention utilise les courants dérivés circulant entre les électrodes actives 212, 222 préférentiellement entre les deux électrodes actives 212, 222. Ces courants dérivés sont classiquement réduits, voire empêchés par les dispositifs de l'état de la technique.

Avantageusement, chaque électrode active 212, 222 est respectivement connectée à un générateur de tension sinusoïdale 210, 220, préférentiellement par un canal respectif. Préférentiellement, le dispositif ne comprend pas de commutateur destiné à faire passer un premier signal d'un générateur de tension 210 aux deux électrodes actives ou un deuxième signal d'un deuxième générateur de tension 210 aux deux électrodes actives. Avantageusement, le dispositif ne comprend pas d'organe de mixage destiné à combiner le premier signal du premier générateur et le deuxième signal du deuxième générateur.

Préférentiellement, le signal produit par chaque générateur est appliqué à une électrode active.

Le dispositif selon l'invention est configuré pour générer un potentiel différent à chaque électrode 212, 222 de sorte à assurer la transmission du courant entre deux électrodes 212, 222, 240 les plus près.

Selon le présent enseignement, le neutre ou point commun flottant à toutes les électrodes actives 212, 222, est connecté ou non à une électrode de retour 240 appliqué sur le corps de l'utilisateur 10 en fonction de la configuration.

Le procédé mettant en œuvre le dispositif génère selon la première configuration une tension composée 110 entre les électrodes actives 212, 222 et/ou selon la deuxième configuration des tensions simples 120, 121 entre les électrodes actives 212, 222 et l'électrode de retour 240 appliquée sur le corps de l'utilisateur 10.

Selon un mode de réalisation préféré, le dispositif est destiné à l'électrothérapie et plus précisément à la diathermie. À cet effet, chaque générateur de tension 210, 220 est configuré pour générer une tension haute fréquence. La tension haute fréquence est préférentiellement comprise entre 100 kHz et 10 MHz.

Avantageusement, le dispositif comprend pour chaque générateur de tension 210, 220 un organe de mesure 213, 223. L'organe de mesure 213, 223 est configuré pour mesurer les paramètres du signal en sortie du générateur de tension 210, 220 isolé.

Préférentiellement, le dispositif comprend pour chaque générateur de tension 210, 220 un module de contrôle 214, 224. Le module de contrôle 214, 224 est configuré pour contrôler le signal en sortie du générateur de tension 210, 220 isolé. Préférentiellement, le module de contrôle 214, 224, régule par exemple la fréquence du courant et/ou le déphasage du générateur de tension. À titre d'exemple, le module de contrôle 214, 224 est un microcontrôleur ou un microprocesseur ou un circuit logique programmable (CPLD Complex Programmable Logic Device) ou un réseau de portes programmables in situ (Field Programmable Gate Array FPGA) ou un circuit analogique.

Avantageusement, l'organe de mesure 213, 223 est configuré pour communiquer avec le module de contrôle 214, 224 de sorte à lui fournir les données nécessaires au contrôle.

Préférentiellement, le dispositif comprend un organe de synchronisation 230 avantageusement agencé de sorte à contrôler les générateurs de tensions 210,220. L'organe de synchronisation 230 est agencé sur une entrée de synchronisation de chaque générateur de tensions 210,220. L'organe de synchronisation 230 est configuré pour permettre la synchronisation ou la désynchronisation des générateurs de tension 210,220 et donc des signaux émis. On entend par synchronisation, que les signaux émis sont à une même pulsation et/ou même fréquence et/ou même période. L'organe de synchronisation 230 génère par exemple un signal de synchronisation pour chaque générateur de sorte à contrôler la synchronisation ou désynchronisation des signaux. Comme illustré aux figures 4 et 5, lors de la synchronisation des deux générateurs de tension 210, 220, les courants circulent entre chaque l'électrode active 212, 222 et l'électrode neutre 240 tandis que lors de la désynchronisation des deux générateurs de tension 210, 220, les courants circulent également entre les deux électrodes actives 212, 220, c'est le courant dérivé 110. Le contrôle de la synchronisation ou désynchronisation des signaux participe à la formation et au maintien de courant dérivé entre les deux électrodes actives.

Avantageusement, les générateurs de tension 210, 220 sont indépendants et peuvent notamment régler le signal de sortie en fonction de l'impédance constatée par le dispositif. Le dispositif selon l'invention est avantageusement configuré pour être un dispositif multi sortie, c'est-à-dire comprenant deux électrodes actives connectées à deux générateurs de tension générant des signaux de sortie distincts.

Avantageusement, le signal émit pour chaque générateur de tension 210, 220 est sinusoïdal. Préférentiellement, V = Vamp x sin (wt + Φ) avec Vamp = Amplitude du signal sinusoïdale, w : fréquence angulaire, Φ : déphasage en degré.

Selon un mode de réalisation préféré, le dispositif est configuré pour permettre le phasage ou le déphasage des signaux de chaque générateur de tension 210, 220. Ainsi, la tension de sortie varie en fonction du déphasage des ondes. Lorsque les signaux des générateurs de tension 210, 220 sont en phase, la tension entre deux électrodes actives 212, 222 est deux fois la tension de sortie Vₒᵤₜ. (V₁₁₀ = 2 x Vₒᵤₜ) avec Vout: tension de sortie du générateur de tension. Lorsque les signaux des générateurs de tension 210, 220 sont déphasés à 180° alors la tension de sortie est nulle, Vₐₘₚ = 0V avec Vamp : Amplitude du signal sinusoïdal de sortie.

Selon le premier mode de réalisation dans sa première configuration, le dispositif selon l'invention est illustré à la figure 1.

Selon cette première configuration de ce premier mode de réalisation, N est égal à deux, avec deux électrodes actives 212, 222 respectivement chacune connectée à un générateur de tension 210, 220. Le dispositif selon cette première configuration ne comporte pas d'électrode de retour. Dans cette première configuration, les générateurs de tension 210, 220 sont agencés en série.

Les générateurs de tensions 210, 220 sont avantageusement isolés grâce à un transformateur 211, 222 respectivement agencés en sortie de chaque générateur de tension 210, 220.

Les transformateurs 211, 222 sont avantageusement configurés pour être en phase ou en déphasage notamment en opposition selon le besoin que les signaux soient en phase ou en déphasage.

Le dispositif selon ce premier mode de réalisation comprend deux organes de mesure 213, 223 agencés en sortie de chaque générateur de tension 210, 220. Chaque organe de mesure 213, 223 est connecté au module de contrôle 214, 224 respectif de chaque générateur de tensions 210, 220.

Dans ce mode de réalisation suivant cette première configuration, en l'absence de l'électrode de retour 240, le courant circulant est le courant dérivé 110 entre les deux électrodes actives 212, 222 appliquées sur le corps d'un utilisateur 10 comme illustré à la figure 3.

Dans cette première configuration, le signal est dit composé :
V₁₁₀=V₁₂₀ + V₁₂₁. Dans lequel V₁₁₀ est la tension du courant 110 circulant entre les 2 électrodes actives 212, 222, V₁₂₀ est la tension du courant 120 circulant entre une électrode active 212 et une électrode neutre 240 non présente, V₁₂₁ est la tension du courant 121 circulant entre une électrode active 222 et une électrode neutre 240 non présente.

Dans cette première configuration, la mise en série des deux générateurs de tensions isolés 210, 220 permet l'augmentation de la tension jusqu'à 2 fois sa valeur nominale pour rechercher l'effet de claquage de l'isolant, c'est-à-dire une augmentation de la perméabilité des tissus biologiques durs (os, ligaments...) et que le courant y circule.

Cette première configuration permet de proposer un traitement par diathermie sérialisée. Cette configuration permet de mettre en série les deux générateurs de tensions 210, 220 et ainsi d'appliquer des signaux générant une diathermie. Ce traitement est réalisé par deux électrodes actives et l'électrode de retour est dite flottante car cette dernière n'est pas appliquée sur l'utilisateur. Cette disposition augmente la tension et non son intensité afin que le courant maximise la conductivité des tissus biologiques et donc l'accélération de la cicatrisation, tout en minimisant leur échauffement. Le but est de favoriser le métabolisme cellulaire desdits tissus avec des tensions pouvant aller jusqu'à 800 Vrms.

Dans cette première configuration, les générateurs de tensions 210, 220 sont configurés pour que les signaux émis soient en déphasage, et synchrones.

Selon ce premier mode de réalisation d'invention, le dispositif peut prendre alternativement une deuxième configuration illustrée à la figure 3.

Selon cette deuxième configuration de ce mode de réalisation, N est égal à trois, avec deux électrodes actives 212, 222 chacune respectivement connectée à un générateur de tension 210, 220. Le dispositif selon cette deuxième configuration comprend une électrode de retour 240. L'électrode de retour 240 est avantageusement connectée au point commun des générateurs de tensions 210, 220. Dans cette deuxième configuration, les générateurs de tensions 210, 220 sont agencés en parallèle.

Dans ce mode de réalisation, suivant cette deuxième configuration comprenant une électrode de retour 240, le courant circule entre les électrodes actives 212, 222 et une électrode de retour 240, mais également avantageusement entre les deux électrodes actives 212, 222 comme illustré à la figure 4.

Dans cette deuxième configuration, le signal est dit simple avec le courant 120, 121. Chaque générateur de tensions 210, 220 alimente une impédance différente. Les électrodes étant appliquées sur le corps de l'utilisateur 10, une impédance existe entre les deux électrodes actives 212, 222. Une tension, V₁₁₀, s'applique entre les 2 électrodes actives 212, 222. La tension V₁₁₀ varie en fonction de la proximité des deux électrodes actives 212, 222 et du déphasage des signaux comme indiqué ci-dessus.

Dans cette deuxième configuration, la mise en parallèle des deux générateurs de tensions 210, 220 permet l'augmentation de l'intensité jusqu'à 2 fois sa valeur nominale pour rechercher l'échauffement des tissus traversés, c'est-à-dire en d'autres termes une action de diathermie. Le rapprochement ou l'éloignement des électrodes actives 212, 222 permet de faire varier la puissance.

Cette deuxième configuration permet de proposer un traitement par diathermie et conductivité combinées. Cette configuration permet de mettre en parallèle les deux générateurs de tensions 210, 220 et ainsi d'appliquer des signaux générant une diathermie et une conductivité. Dans cette deuxième configuration, l'électrode de retour 240 est positionnée sur le corps de l'utilisateur 10 afin de créer trois segments de courants répartis entre les trois électrodes. La somme des trois segments de courants 110, 120, 121, correspond à la puissance délivrée par le dispositif d'électrothérapie. De plus, cette configuration augmente l'intensité et non la tension, ce qui favorise l'échauffement (diathermie) des tissus biologiques traversés avec des intensités pouvant aller jusqu'à 4 ampères.

En figures 4 et 5, les deux électrodes actives 212, 222 transmettent un courant sinusoïdal dont la fréquence et les caractéristiques peuvent être différentes. Elles convergent vers une électrode de retour 240 via des segments électriques où circule le courant 120 et où circule le courant 121 avec la possibilité de créer un troisième segment électrique où circule le courant 110 selon les réglages de l'électrode de retour 240. Le dispositif correspondant permet de répartir la puissance émise d'un dispositif biphasé par le biais de 3 segments électriques de trois électrodes 212, 222, 240 sur le corps de l'utilisateur 10. Entre chaque électrode 212, 222, 240 existe un segment électrique et le praticien peut définir comment répartir la puissance entre ces 3 électrodes 212, 222, 240. Le dispositif est configuré pour synchroniser ou désynchroniser via le déphasage des signaux.

En figure 4, les signaux sont synchronisés et peuvent être en phase ou en déphasage. Il n'y a pas de courant dérivé.

En figure 5, les signaux sont désynchronisés et peuvent être en phase ou en déphasage. Il y a un courant dérivé.

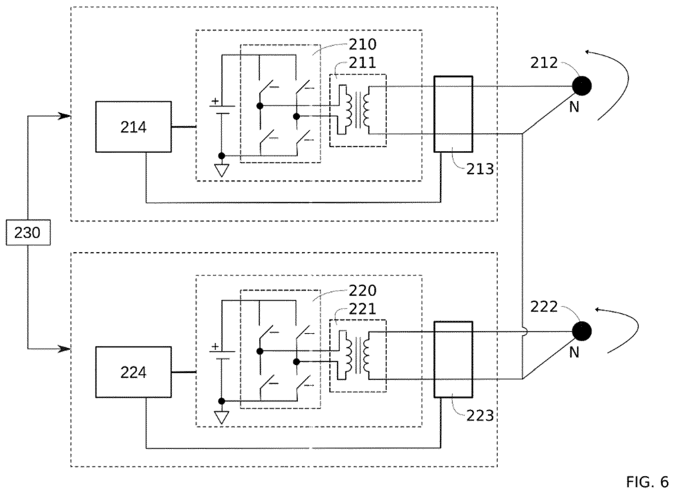

Selon un deuxième mode de réalisation, illustré à la figure 6, N est égal à deux, avec deux électrodes actives 212, 222 respectivement chacune connectée à un générateur de tension 210, 220. Le dispositif selon ce deuxième mode de réalisation ne comporte pas d'électrode de retour. Dans cette première configuration, les générateurs de tension 210, 220 sont agencés en série.

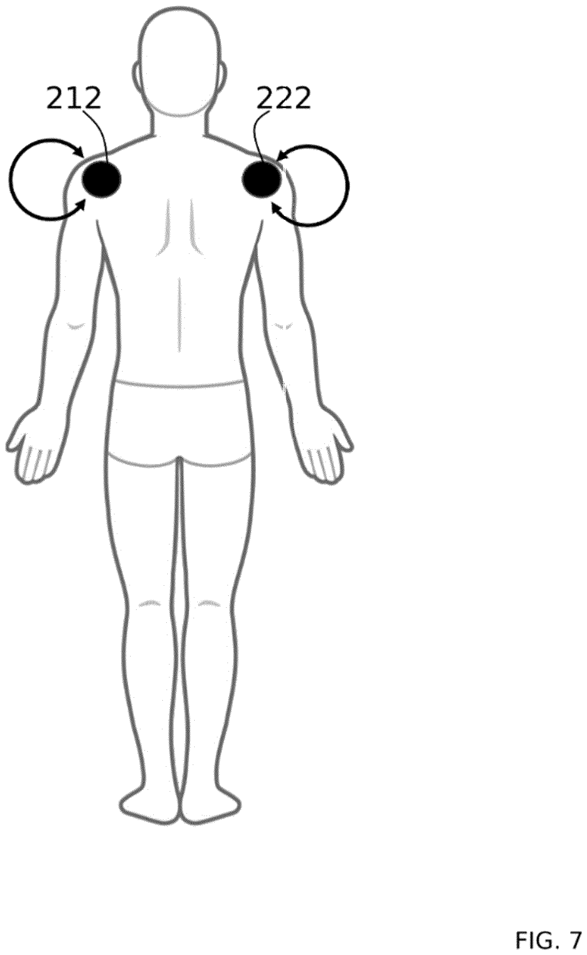

Les électrodes actives 212, 222 sont avantageusement configurées pour être simultanément ou alternativement actives et neutres comme illustré à la figure 6 et à la figure 7 pour assurer une circulation du courant en sortie et en entrée sur une seule électrode 212, 222.

Selon un mode de réalisation, les électrodes actives peuvent être intelligentes. Elles sont équipées d'une centrale inertielle qui permet de connaitre en temps réel le mouvement du praticien. Le praticien pourra modifier son traitement en cours d'utilisation pour une meilleure efficacité. Elle reconnaitra aussi le type d'électrode utilisée. Le module de contrôle 214, 224 adapte la sortie en fonction du type de l'électrode détecté.

A titre d'exemple, le dispositif est avantageusement prévu pour une tension simple maximale de 400 Vrms, un courant maximal simple de 1.2Arms et une puissance de 150 W sur des fréquences allant de 300 kHz à 1 MHz et une tension composée maximale de 800 Vrms, un courant maximal simple de 2.4Arms et une puissance de 300 W sur des fréquences allant de 300 kHz à 1 MHz.

L'invention permet donc avantageusement de varier la tension et l'intensité des courants hautes fréquences et de travailler sur des intensités et/ou des tensions et/ou des fréquences différentes et de combiner ou non différents types d'électrodes.

Selon une possibilité, les générateurs de tensions 210, 220, peuvent fonctionner pour produire des tensions multi fréquences par modulation des signaux basse fréquence, moyenne fréquence haute fréquence comme décrits dans une invention du demandeur. Le générateur de tension à la fréquence la plus forte est modulé en amplitude par le générateur de tension à la fréquence la plus faible. Les puissances sont déséquilibrées. Chaque générateur doit fournir une puissance correspondant à ses électrodes et à celui des électrodes associées. La puissance est réglée en fonction de l'amplitude et du déphasage de chaque générateur.

Selon cette possibilité, le dispositif d'électrothérapie comprend un premier générateur de tension configuré pour générer une première tension ayant une première fréquence et comprenant une première borne de raccordement et une deuxième borne de raccordement, et un deuxième générateur de tension configuré pour générer une deuxième tension ayant une deuxième fréquence strictement supérieure à la première fréquence et comprenant une troisième borne de raccordement et une quatrième borne de raccordement, caractérisé en ce qu'il comprend un canal d'émission, un canal de réception, et que la première borne de raccordement et la troisième borne de raccordement sont reliées audit canal d'émission et que la deuxième borne de raccordement et la quatrième borne de raccordement sont reliées audit canal de réception.

Cette possibilité permet d'appliquer deux tensions de deux fréquences différentes par un seul et même canal d'émission et un seul et même canal de réception.

Plus particulièrement, le dispositif d'électrothérapie comprend une unité de commande configurée pour commander le premier générateur de tension pour générer la première tension à une première fréquence, commander le deuxième générateur de tension la deuxième tension à une deuxième fréquence de manière simultanée.

Avantageusement, l'unité de commande est configurée pour générer dans le canal d'émission, un signal associant une tension sinusoïdale d'une première fréquence modulée par une troisième fréquence et associée à une tension sinusoïdale d'une deuxième fréquence modulée par une quatrième fréquence, le signal étant la somme de la tension du premier module de générateur et de la tension du deuxième module de générateur.

Un autre aspect concerne un procédé pour le fonctionnement d'un dispositif d'électrothérapie tel que décrit pour cette possibilité comprenant :
a. la génération d'une première tension à une première fréquence par le premier générateur, et
b. la génération d'une deuxième tension à une deuxième fréquence strictement supérieure à la première fréquence par le deuxième générateur, les étapes a et b étant simultanées. Ainsi, les deux générateurs de tensions fonctionnent simultanément.

Selon un exemple, le premier générateur de tension est configuré pour générer une tension sinusoïdale.

Selon un exemple, la première fréquence est comprise dans un premier intervalle de fréquence entre 1 kHz à 10 kHz.

Selon un exemple, le premier générateur de tension est configuré pour générer une tension sinusoïdale de première fréquence modulée par une troisième fréquence.

Selon un exemple, la troisième fréquence est comprise dans un troisième intervalle de fréquence compris entre 1 Hz et 150 Hz.

Selon un exemple, la troisième fréquence est une tension sinusoïdale.

Cette possibilité du dispositif propose de créer un signal sinusoïdal de Moyenne Fréquence afin de moduler le signal de Basse Fréquence (électrostimulation) et de le combiner avec un signal sinusoïdal de Haute Fréquence (diathermie). Le dispositif selon cette possibilité permet de combiner au sein d'un unique et même canal les vertus de l'électrostimulation et de la radiofréquence générant la diathermie. Le dispositif selon cette possibilité produit un courant non invasif stimulant les mécanismes de cicatrisation naturels du corps et favorisant l'échange cellulaire. Elle offre d'excellents résultats de réadaptation grâce à une récupération rapide des fonctions musculaires et articulaires.

Le résultat est un courant sinusoïdal de 1 kHz à 10 kHz modulé à une fréquence de 1 Hz à 150 Hz. En électrostimulation, la modulation du courant stimulateur permet d'éviter la tétanie des muscles excités.

Selon un exemple, le deuxième générateur de tension est configuré pour générer une tension sinusoïdale.

Selon un exemple, la deuxième fréquence est comprise dans un deuxième intervalle de fréquence compris entre 100 kHz à 10 MHz.

Selon un exemple, le deuxième générateur de tension est configuré pour générer une tension sinusoïdale de deuxième fréquence modulée par une quatrième fréquence.

Selon un exemple, la quatrième fréquence est comprise dans un quatrième intervalle de fréquence compris entre 1 Hz et 150 Hz.

Selon un exemple, la quatrième fréquence est à impulsion.

Selon un exemple, le deuxième de générateur de tension comprend une commande d'activation configurée pour générer une tension sinusoïdale à impulsion à la quatrième fréquence.

Selon un exemple, le premier de générateur de tension comprend une commande d'émission configurée pour émettre la tension du premier module de générateur à la quatrième fréquence.

L'invention est définie dans les revendications suivantes.

### Liste des références

- 10.: Corps de l'utilisateur
- 110.: Courant dérivé entre deux électrodes actives
- 111.: Courant dérivé entre deux électrodes actives
- 112.: Courant dérivé entre deux électrodes actives
- 120.: Courant dérivé entre une électrode active et une électrode neutre
- 121.: Courant dérivé entre une électrode active et une électrode neutre
- 122.: Courant dérivé entre une électrode active et une électrode neutre
- 210.: Générateur de tension
- 211.: Transformateur
- 212.: Électrode active reliée au premier générateur de tension
- 213.: Organe de mesure
- 214.: Module de contrôle
- 220.: Générateur de tension
- 221.: Transformateur
- 222.: Électrode active reliée au deuxième générateur de tension
- 223.: Organe de mesure
- 224.: Module de contrôle
- 230.: Organe de synchronisation
- 240.: Électrode neutre ou de retour

## Revendications

1. Dispositif d'électrothérapie apte à fournir un traitement par diathermie et/ou conductivité au corps d'un utilisateur, ledit dispositif comprenant
- N électrodes (212, 222, 240) configurées pour être appliquées au corps de l'utilisateur, N étant égal à 2 ou 3,
- deux générateurs de tension sinusoïdale (210, 220),
- un transformateur (211, 221) pour chaque générateur de tension de sorte que les deux générateurs de tension (210, 220) sont isolés par un transformateur (211, 221) et
- une unité de commande configurée pour que le dispositif prenne alternativement:
• une première configuration dite en série dans laquelle les N égal à 2 électrodes (212, 222) étant des électrodes actives et les deux générateurs de tension (210, 220) sont mis en série, le dispositif ne comprend pas d'électrode de retour (240), chaque électrode active (212, 222) étant connectée à un générateur de tension (210, 220) le dispositif est configuré pour générer un potentiel différent à chaque électrode active (212, 222) de sorte à assurer la transmission du courant entre deux électrodes actives (212, 222) les plus près,
• une deuxième configuration dite en parallèle dans laquelle N égale à 3, dont deux électrodes (212, 220) sont des électrodes actives chacune respectivement connectée à un générateur de tension (210, 220) et une électrode (240) est une électrode de retour pour former la masse et est connectée à chaque générateur de tension (210, 220), les deux générateurs de tension (210, 220) sont mis en parallèle, le dispositif est configuré pour générer un potentiel différent à chaque électrode active (210, 222) de sorte à assurer la transmission du courant entre deux électrodes actives les plus près et/ou entre chaque électrode active (212, 222) et l'électrode de retour (240).

2. Dispositif selon la revendication précédente dans lequel chaque générateur de tension (210, 220) est configuré pour générer une tension haute fréquence, préférentiellement entre 100 kHz et 10 MHz.

3. Dispositif selon l'une quelconque des revendications précédentes dans lequel chaque électrode active (210, 222) est connectée à un générateur de tension (210, 220) de sorte à recevoir respectivement un premier signal ou un deuxième signal produit respectivement par un générateur de tension (210, 220), préférentiellement, le premier signal et le deuxième signal sont différents.

4. Dispositif selon l'une quelconque des revendications précédentes dans lequel les électrodes actives (212, 222) sont configurées pour être mobiles.

5. Dispositif selon l'une quelconque des revendications précédentes dans lequel les électrodes actives (212, 222) sont capacitives ou résistives ou multipolaires.

6. Dispositif selon l'une quelconque des revendications précédentes dans lequel chaque générateur de tension (210, 220) comprend un organe de mesure (213, 223) configuré pour mesurer des paramètres de sortie du générateur de tension (210, 220).

7. Dispositif selon l'une quelconque des revendications précédentes dans lequel chaque générateur de tension (210, 220) comprend un module de contrôle (214, 224) configuré pour contrôler le courant de sortie et/ou la fréquence et/ou le déphasage du générateur de tension (210, 220).

8. Dispositif selon les deux revendications précédentes dans lequel l'organe de mesure (213, 223) communique avec le module de contrôle (214, 224) pour lui fournir des données nécessaires au contrôle.

9. Dispositif selon une quelconque des revendications précédentes comprenant un organe de synchronisation (230) configuré pour contrôler la synchronisation ou la désynchronisation des générateurs de tension (210, 220).

10. Dispositif selon la revendication précédente dans lequel dans la première configuration, les signaux émis par les générateurs de tension (210, 220) sont en déphasage et synchrones.

11. Dispositif selon l'une quelconque des revendications précédentes dans lequel :
• le premier générateur de tension (210) est configuré pour générer une première tension ayant une première fréquence et comprenant une première borne de raccordement et une deuxième borne de raccordement,
• le deuxième générateur de tension (220) est configuré pour générer une deuxième tension ayant une deuxième fréquence strictement supérieure à la première fréquence et comprenant une troisième borne de raccordement et une quatrième borne de raccordement,
• un canal d'émission,
• un canal de réception,
Et la première borne de raccordement et la troisième borne de raccordement sont reliées audit canal d'émission et que la deuxième borne de raccordement et la quatrième borne de raccordement sont reliées audit canal de réception,
• une unité de commande configurée pour simultanément :
- commander le premier générateur de tension (210) pour générer la première tension à une première fréquence,
- commander le deuxième générateur de tension (220) la deuxième tension à une deuxième fréquence,
l'unité de commande étant configurée pour générer dans le canal d'émission, un signal associant une tension sinusoïdale à la première fréquence modulée par la troisième fréquence et associé à une tension sinusoïdale à la deuxième fréquence modulée par la quatrième fréquence, le signal étant la somme de la tension du premier de générateur et de la tension du deuxième de générateur.

## Patentansprüche

1. Elektrotherapievorrichtung, die dazu geeignet ist, eine Diathermie- und/oder Leitfähigkeitsbehandlung an dem Körper eines Benutzers bereitzustellen, wobei die Vorrichtung Folgendes umfasst:
- N Elektroden (212, 222, 240), die so konfiguriert sind, dass sie an dem Körper des Benutzers angebracht werden, wobei N gleich 2 oder 3 ist,
- zwei Sinusspannungsgeneratoren (210, 220),
- einen Transformator (211, 221) für jeden Spannungsgenerator, so dass die beiden Spannungsgeneratoren (210, 220) durch einen Transformator (211, 221) isoliert sind, und
- eine Steuereinheit, die so konfiguriert ist, dass die Vorrichtung abwechselnd Folgendes annimmt:
• eine erste Reihenkonfiguration, wobei die N gleich 2 Elektroden (212, 222) aktive Elektroden sind und die beiden Spannungsgeneratoren (210, 220) in Reihe geschaltet sind, wobei die Vorrichtung keine Rückelektrode (240) umfasst, wobei jede aktive Elektrode (212, 222) mit einem Spannungsgenerator (210, 220) verbunden ist, wobei die Vorrichtung so konfiguriert ist, dass sie an jeder aktiven Elektrode (212, 222) ein anderes Potenzial erzeugt, so dass die Stromübertragung zwischen zwei am nächsten beieinander liegenden aktiven Elektroden (212, 222) sichergestellt wird,
• eine zweite Parallelkonfiguration, wobei N gleich 3 ist, wobei zwei Elektroden (212, 220) davon aktive Elektroden sind, die jeweils mit einem Spannungsgenerator (210, 220) verbunden sind, und eine Elektrode (240) eine Rückelektrode ist, um die Masse zu bilden, und mit jedem Spannungsgenerator (210, 220) verbunden ist, wobei die beiden Spannungsgeneratoren (210, 220) parallel geschaltet sind, wobei die Vorrichtung so konfiguriert ist, dass sie an jeder aktiven Elektrode (210, 222) ein anderes Potenzial erzeugt, so dass die Stromübertragung zwischen zwei am nächsten beieinander liegenden aktiven Elektroden und/oder zwischen jeder aktiven Elektrode (212, 222) und der Rückelektrode (240) sichergestellt wird.

2. Vorrichtung nach dem vorhergehenden Anspruch, wobei jeder Spannungsgenerator (210, 220) so konfiguriert ist, dass er eine Hochfrequenzspannung, vorzugsweise zwischen 100 kHz und 10 MHz, erzeugt.

3. Vorrichtung nach einem der vorhergehenden Ansprüche, wobei jede aktive Elektrode (210, 222) mit einem Spannungsgenerator (210, 220) verbunden ist, so dass sie jeweils ein erstes Signal oder ein zweites Signal empfängt, das von einem Spannungsgenerator (210, 220) erzeugt wird, wobei das erste Signal und das zweite Signal vorzugsweise unterschiedlich sind.

4. Vorrichtung nach einem der vorhergehenden Ansprüche, wobei die aktiven Elektroden (212, 222) so konfiguriert sind, dass sie beweglich sind.

5. Vorrichtung nach einem der vorhergehenden Ansprüche, wobei die aktiven Elektroden (212, 222) kapazitiv oder resistiv oder mehrpolig sind.

6. Vorrichtung nach einem der vorhergehenden Ansprüche, wobei jeder Spannungsgenerator (210, 220) ein Messelement (213, 223) umfasst, das so konfiguriert ist, dass es Ausgangsparameter des Spannungsgenerators (210, 220) misst.

7. Vorrichtung nach einem der vorhergehenden Ansprüche, wobei jeder Spannungsgenerator (210, 220) ein Steuermodul (214, 224) umfasst, das so konfiguriert ist, dass es den Ausgangsstrom und/oder die Frequenz und/oder die Phasenverschiebung des Spannungsgenerators (210, 220) steuert.

8. Vorrichtung nach den beiden vorhergehenden Ansprüchen, wobei das Messelement (213, 223) mit dem Steuermodul (214, 224) in Verbindung steht, um ihm die für die Steuerung erforderlichen Daten zu bereitzustellen.

9. Vorrichtung nach einem der vorhergehenden Ansprüche, die ein Synchronisationselement (230) umfasst, das so konfiguriert ist, dass es die Synchronisation oder Desynchronisation der Spannungsgeneratoren (210, 220) steuert.

10. Vorrichtung nach dem vorhergehenden Anspruch, wobei in der ersten Konfiguration die von den Spannungsgeneratoren (210, 220) gesendeten Signale phasenversetzt und synchron sind.

11. Vorrichtung nach einem der vorhergehenden Ansprüche, wobei:
• der erste Spannungsgenerator (210) so konfiguriert ist, dass er eine erste Spannung erzeugt, die eine erste Frequenz aufweist, und eine erste Anschlussklemme und eine zweite Anschlussklemme umfasst,
• der zweite Spannungsgenerator (220) so konfiguriert ist, dass er eine zweite Spannung erzeugt, die eine zweite Frequenz aufweist, die strikt über der ersten Frequenz liegt, und eine dritte Anschlussklemme und eine vierte Anschlussklemme umfasst,
• einen Sendekanal,
• einen Empfangskanal,
und wobei die erste Anschlussklemme und die dritte Anschlussklemme mit dem Sendekanal verbunden sind und die zweite Anschlussklemme und die vierte Anschlussklemme mit dem Empfangskanal verbunden sind,
• eine Steuereinheit, die gleichzeitig für Folgendes konfiguriert ist:
- Steuern des ersten Spannungsgenerators (210), um die erste Spannung bei einer ersten Frequenz zu erzeugen,
- Steuern des zweiten Spannungsgenerators (220) die zweite Spannung mit einer zweiten Frequenz,
wobei die Steuereinheit so konfiguriert ist, dass sie im Sendekanal ein Signal erzeugt, das einer Sinusspannung mit der ersten Frequenz, moduliert durch die dritte Frequenz, und einer Sinusspannung mit der zweiten Frequenz, moduliert durch die vierte Frequenz, zugeordnet ist, wobei das Signal die Summe der Spannung des ersten Generators und der Spannung des zweiten Generators ist.

## Claims

1. Electrotherapy device able to deliver diathermy and/or conductivity treatment to the body of a user, said device comprising
- N electrodes (212, 222, 240) configured to be applied to the body of the user, N being equal to 2 or 3,
- two sinusoidal voltage generators (210, 220),
- a transformer (211, 221) for each voltage generator such that the two voltage generators (210, 220) are isolated by a transformer (211, 221), and
- a control unit configured so that the device alternatively takes:
• a first so-called series configuration in which, the N equal to 2 electrodes (212, 222) being active electrodes and the two voltage generators (210, 220) are connected in series, the device does not comprise a return electrode (240), each active electrode (212, 222) being connected to a voltage generator (210, 220) the device is configured to generate a different potential at each active electrode (212, 222) so as to ensure the transmission of current between the two closest active electrodes (212, 222),
• a second so-called parallel configuration, in which N equals 3, two electrodes (212, 220) of which are active electrodes each respectively connected to a voltage generator (210, 220), and an electrode (240) of which is a return electrode to form the ground and is connected to each voltage generator (210, 220), the two voltage generators (210, 220) are connected in parallel, the device is configured to generate a different potential at each active electrode (210, 222) so as to ensure the transmission of current between the two nearest active electrodes and/or between each active electrode (212, 222) and the return electrode (240).

2. Device according to the preceding claim, wherein each voltage generator (210, 220) is configured to generate a high-frequency voltage, preferably between 100 kHz and 10 MHz.

3. Device according to either one of the preceding claims, wherein each active electrode (210, 222) is connected to a voltage generator (210, 220) to receive respectively a first signal or a second signal produced respectively by a voltage generator (210, 220), preferentially the first signal and the second signal are different.

4. Device according to any one of the preceding claims, wherein the active electrodes (212, 222) are configured to be movable.

5. Device according to any of the preceding claims, wherein the active electrodes (212, 222) are capacitive, resistive or multipole.

6. Device according to any one of the preceding claims, in which each voltage generator (210, 220) comprises a measuring member (213, 223) configured to measure the output parameters of the voltage generator (210, 220).

7. Device according to any one of the preceding claims, wherein each voltage generator (210, 220) comprises a control module (214, 224) figured to control the output current and/or the frequency and/or the phase shift of the voltage generator (210, 220).

8. Device according to the preceding two claims, wherein the measuring member (213, 223) communicates with the control module (214, 224) to provide it with data necessary for control.

9. Device according to any one of the preceding claims, comprising a synchronisation member (230) configured to control the synchronisation or desynchronisation of the voltage generators (210, 220).

10. Device according to the preceding claim, wherein, in the first configuration, the signals emitted by the voltage generators (210, 220) are phase-shifted and synchronous.

11. Device according to any one of the preceding claims, wherein
• the first voltage generator (210) is configured to generate a first voltage having a first frequency and comprising a first connection terminal and a second connection terminal,
• the second voltage generator (220) is configured to generate a second voltage having a second frequency strictly higher than the first frequency and comprising a third connection terminal and a fourth connection terminal,
• a transmission channel,
• a reception channel,
and the first connection terminal and the third connection terminal are connected to said transmission channel, and the second connection terminal and the fourth connection terminal are connected to said reception channel,
• a control unit configured to simultaneously:
- control the first voltage generator (210) to generate the first voltage at a first frequency,
- control the second voltage generator (220) the second voltage at a second frequency,
the control unit being configured to generate in the transmission channel a signal associating a sinusoidal voltage at the first frequency modulated by the third frequency and associated with a sinusoidal voltage at the second frequency modulated by the fourth frequency, the signal being the sum of the voltage from the first generator and the voltage from the second generator.
